# EUROPEAN PATENT APPLICATION

(11) **EP 1 467 299 A2**
(43) Date of publication of application: **13.10.2004**
(21) Application number: 04007338.9
(22) Date of filing: 26.03.2004
(51) Int. Cl.: G06F 17/60

(54) **Methods and structure for automated active pharmaceuticals development**

(30) Priority: 28.03.2003 US 458617 P
(71) Applicant: Solutia Inc., St. Louis, Missouri 63141 (US)
(72) Inventor: Fischer, Alan Eric, Biberstein CH-5023 (CH); Lacy, Ronald M., Snoqualmie Washington 98065 (US)
(74) Representative: Albrecht, Thomas, Dr.

(57) **Abstract**

Methods and associated structures for integrating molecule database information and chemical synthesis database information to generate a project synthesis plan shared by multiple entities within a chemical development service provider enterprise. The shared information is used by various management, R&D and financial entities within such an enterprise. Shared use of common data improves accuracy of the data used by each such entity and reduces need for error-prone human intervention in generating and manipulating relevant data.

## Description

### CROSS REFERENCE

This application claims the benefit of United States Provisional Patent Application Serial No. 60/458,817, filed March 28, 2003 and entitled Methods And Structure For Automated Active Pharmaceuticals Development.

### BACKGROUND OF THE INVENTION

The invention relates broadly to chemical compound development processes and more specifically relates to methods and structures for integrating numerous aspects of management of chemical compound development processes such as within a service organization manufacturing pharmaceutical compounds.

In a number of chemical development industries and applications, the development of complex chemical compounds entails a number of project management goals and objectives. For example, scientific management requires documentation of materials, processes and activities involved in development of a particular compound so that the resultant compound may be reliably reproduced. Financial management of such projects may require information regarding costs including human resources, as well as raw materials consumed by the compound development project. Other financial management aspects of project developments relate to purchasing and inventory of raw materials within the development facility.

In the pharmaceuticals sector of the chemical industries, the problems associated with project management for chemical compound development are often more acute. Often chemical development service providers are contracted by pharmaceutical companies to identify cost-effective procedures for reliable, repeatable manufacture of desired compounds. Such chemical development service providers often work with narrow profit margins such that cost control for development of a new compound is critical to profitable success of a project. Further, in the pharmaceuticals industry, quality control in the repeated manufacture of desired chemical compounds may be critical to the safety and usability of the chemical compound. Still further, in pharmaceutical applications of chemical compounds, regulatory aspects of the industry require substantial documentation and reporting on the process and materials involved in manufacture of chemical compounds.

It is an ongoing problem for such chemical compound development service providers to document and manage all aspects of their business including, financial, materials, scientific and process documentation, etc. Present solutions to these ongoing problems involved multiple disparate systems and methodologies for managing and documenting various aspects of the chemical compound development. Financial management of such projects involves the collection of data with appropriate financial management systems. Scientific and process documentation by the scientists and engineers utilize other data processing tools. Additionally, materials managers control their inventories with processes supported by yet another system.

Such a disparate collection of data and tools leads to significant inefficiencies and errors in the production and management of data relating to services provided by chemical product development service organizations. Duplication of data among the various disparate tools can lead to inconsistencies in the data used for various management aspects of such projects. Inefficiencies associated with duplication of certain data and duplication of management aspects of the project among the various disparate tools can lead to increased complexities and associated costs. Such increased complexity and cost can negatively impact profitability for such chemical compound development service organizations.

The above discussion provides evidence that a need exists for improved gathering, organization and management of data for chemical compound development service organizations.

### SUMMARY OF THE INVENTION

The present invention solves the above and other related problems, thereby advancing the state of the useful arts, by providing methods and associated systems architectures and structures for generating, integrating and sharing data relevant to the management of chemical compound development for pharmaceutical industries. The invention therefore provides for improved utilization of shared data among a plurality of cooperating organizations within the business enterprise of the service organization. More specifically, the methods and structures of the present invention integrate data collection and management for previously disparate data processing needs and users including, for example, scientific data management, standards compliance data management, activity and resource data management, materials data management and financial control and oversight data management. The invention allows for a complete view of a project from multiple management aspects including financial, scientific, time, tasks, and quality assurance.

In some aspects of the invention, data is gathered and associated with a compound synthesis plan. A synthesis plan identifies activities and associated steps in producing a desired chemical compound. Each step of the process is associated with information regarding resources required for performing the step, specific activities to complete the step and materials required in the processing of the step. Cost and price information may also be associated with each activity, resource and material so that financial management may be integrated with the data management of the invention. Other aspects of the invention, mathematical and scientific influences on the chemical and financial/business yields for a given synthesis plan may be factored into project planning. In particular, molecular weights, chemical yields, losses and stoichiometry may be considered in project planning provided by features and aspects of the invention.

As used herein, the terms compound and molecule are used interchangeably and include substances composed of atoms or ions of one or more elements in chemical combination, wherein the constituents are united by bonds or valence forces.

A first feature of the invention therefore provides systems and a method for chemical compound development project management comprising: providing a molecule database such that records of the molecule database include molecular information regarding reactants and products of the synthesis steps required to produce an identified target molecule; providing a synthesis database such that records of the synthesis database include activity information regarding activities to transition between the synthesis steps of the target molecule synthesis; and deriving information regarding a chemical compound development project from the molecule database and from the synthesis database.

Another aspect of the invention further provides that the step of deriving includes: identifying, from the molecule database, materials required in production of the target molecule.

Another aspect of the invention further provides that the step of deriving further includes: generating a bill of materials identifying quantities of all materials required in production of the target molecule.

Another aspect of the invention further provides that the step of deriving includes: identifying, from the synthesis database, resources required in production of the target molecule.

Another aspect of the invention further provides that the step of deriving further includes: identifying, from the synthesis database, activities associated with the identified resources.

Another aspect of the invention further provides that the step of deriving further includes: generating a GANTT chart scheduling the identified resources and the identified activities in production of the target molecule.

Another aspect of the invention further provides that the step of deriving includes: identifying, from the molecule database, materials required in production of the target molecule; identifying, from the synthesis database, resources required in production of the target molecule; identifying, from the synthesis database, costs associated with the identified materials and associated with the identified resources; and generating an estimated project cost from the identified costs.

Another aspect of the invention further provides that the step of deriving includes: searching the molecule database or the synthesis database using chemical indicia to retrieve information regarding a chemical compound development project.

A second feature of the invention provides a system for target chemical compound development comprising: a molecule database including information regarding intermediate chemical compounds required to produce the target chemical compound; a synthesis database including information regarding steps required to transition between the synthesis steps of the target molecule synthesis; and a chemical compound development management system for generating project synthesis plans integrating information from both the molecule database and the synthesis database.

### BRIEF DESCRIPTION OF THE DRAWINGS

Figure 1 is a block diagram of a system in which the features of the present invention are beneficially applied.

Figure 2 is a flowchart describing a method of the present invention to generate and execute a project synthesis plan.

Figure 3 is a flowchart describing a method of the present invention to utilize the shared information in a project synthesis plan for various administrative and managerial purposes.

Figures 4 through 12 are exemplary user display screens for typical interaction between a user and the features of the present invention.

Figure 13 depicts data relationships useful in providing the features of the present invention.

Figures 14 through 20 are additional exemplary user display screens for typical interaction between a user and the features of the present invention.

### DETAILED DESCRIPTION OF THE DRAWINGS

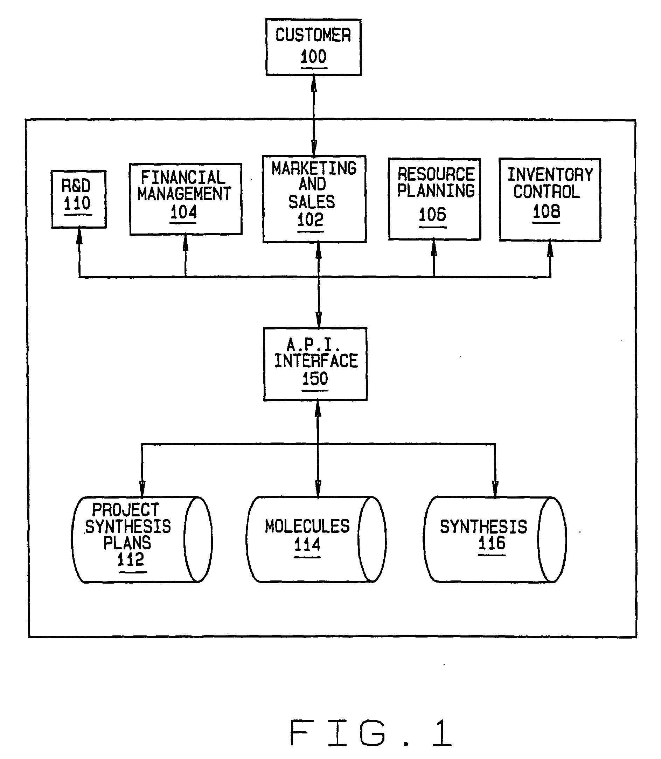

Figure 1 is a block diagram of a typical chemical compound development service provider enterprise 2 beneficially applying features of the present invention. Service provider enterprise 2 interacts with customer 100 in the development and production of customized chemical compounds. For example, customer 100 may be a large pharmaceuticals manufacturing entity contracting with a service vendor 2 to develop materials and procedures appropriate for manufacturing a particular desired chemical compound for pharmaceutical applications. The customer 100 may receive the product provided by the vendor 2 in the form of material, formulae and procedures and scale the processes as required for mass manufacturing of the intended chemical compound.

As noted above, present techniques and systems for management within such a service organization (vendor 2 of figure 1) apply a number of disparate systems and applications for information management. For example, financial management of the service vendor may have totally separate systems and data from the systems and data used by engineering or R&D services within the same enterprise.
Marketing, sales, resource planning and inventory control operations within the enterprise may similarly each have their own unique systems for performing their desired functions.

As shown in figure 1, a service vendor 2 beneficially applying features of the present invention integrates information management for various functions within the enterprise through a common active pharmaceuticals ingredient interface 150 ("API interface"). Various groups and functions within service provider enterprise 2 may share access to common data for reporting and monitoring processes, resources, materials and costs associated with services provided by the enterprise.

For example, in a pharmaceuticals development service provider enterprise, a number of groups within the enterprise may share access to information pertaining to synthesis plans for various projects on behalf of various customers stored in project synthesis plans database 112. Similarly, a number of groups within the pharmaceuticals service provider enterprise may share access to information describing standard molecules or compounds known to the enterprise and stored in molecules database 114. Still further, organizations within the entity may share data regarding synthesis procedures, resources and activities stored in synthesis database 116.

API interface 150 represents management processing to coordinate desired activities by multiple organizations within the enterprise. Coordination of such shared access may be provided by any of a number of standard techniques known to those of ordinary skill the art including, for example, structured file management techniques such as relational or hierarchical database management systems, object oriented database management systems and other database management systems. In one exemplary embodiment of the invention, API interface 150 as well as associated databases may be implemented using the Microsoft .NET framework and associated development tools and run-time support. As another design choice, features and aspects hereof may be implemented in any programming language (such as C++) operable on any computer platform (such as a Windows PC or a UNIX workstation) using other standard, commercially available user interface application programming interfaces. Those of ordinary skill in the art will recognize a wide variety of system and software architectures that may be employed to implement features of the present invention as discussed further herein below.

Further, those of ordinary skill in the art will readily recognize that although project synthesis plans database 112, molecules database 114 and synthesis database 116 are shown in figure 1 as distinct databases, they may be managed within a single database management system and/or physically stored in a common storage medium. In other words, the databases shown may be logically distinguished in terms of the type of information stored therein and the functions that use the data though they need not be physically distinguished in the same manner. Further, storage media used to store the information may be physically coupled to a single computer or may be distributed utilizing well-known distributed computing and storage techniques.

Exemplary groups within vendor enterprise 2 that may share access to such databases include R&D or engineering groups 110, financial management groups 104, marketing and sales organizations 102, resource planning organizations 106 and inventory control organizations 108. In general, a marketing and sales organization 102 within a service vendor enterprise 2 may initiate a new project on behalf of a customer. The marketing and sales organization is typically the direct interface with the customer 100 to receive requests for services from the customer 100. Marketing and sales organization 102 would therefore populate the project synthesis plans database 112 with information regarding the particular new project and/or new client. Specific information pertaining to the particular requested compound may also be entered by the marketing and sales department 102 into the project synthesis plans database 112. Such a project synthesis plan (including customer information) may also incorporate resource requirements and cost information so that the sales and marketing organization 102 may provide a price and schedule quote in response to the customer's request.

When such a quoted project (an "offer") is accepted by the customer, work may commence thereon. Other organizations within service vendor enterprise 2 may share the same project synthesis plan information generated by marketing and sales organization 102 and stored in project synthesis plans database 112. For example, financial management group 104 may analyze data associated with the cost of one or more project synthesis plans to verify profitability and to analyze present and future economic issues associated with the enterprise. R&D group 110 may access the information to provide or review specifications of the customer s requirements in commencing the chemical experimentation and development. Further, R&D group 110 may record information in the project synthesis plans database 112 indicating progress in the chemical development and testing for the newly commenced project. Resource planning group 106 within the service provider enterprise 2 may provide centralized planning for chemical laboratory equipment and production facilities so that multiple projects may be performed concurrently by multiple groups sharing common resources of the enterprise 2. Resource planning organization 106 may therefore share the same information stored in project synthesis plans database 112 to coordinate planning and scheduling of various shared resources within the enterprise. Inventory control group 108 may manage the inventory of raw materials maintained by the enterprise for production of new chemical compounds on behalf of customers. Inventory control group 108 may therefore share information in the project synthesis plans database 112 to anticipate future inventory needs so as to maintain an appropriate flow of raw materials into, and out of the enterprise.

In constructing such a project synthesis plan, the enterprise may refer to information stored in other databases including, for example, molecules database 114 and synthesis database 116. As noted above, molecules database 114 may contain information regarding the various intermediate chemical compounds required to produce a given target chemical compound requested by the customer. As is known in the art, producing a chemical compound often involves a sequence of steps potentially generating a number of intermediate chemical compositions useful in deriving the desired target compound. The molecules database 114 therefore may contain information identifying the raw materials, any intermediate compounds required to generate a given target compound and reactants involved in the chemical process. Still further, the molecules database 114 may incorporate safety information regarding the materials and processes involved in the generation of a desired molecule or any intermediate compounds.

In addition, each step in the process of constructing a desired target chemical compound may involve a sequence of activities and resources required to achieve the desired target chemical compound or intermediate compound. Synthesis database 116 therefore may store information regarding activities and resources associated with the chemistry required to transition from a first chemical compound in the required sequence to a second chemical compound. Synthesis database 116 may also include stoichiometry information as well as information regarding isolation and sampling losses. Such information may be collectively referred to as a chemical recipe.

The combination of information stored in molecules database 114 and in synthesis database 116 therefore may describe the materials, activities and resources required to start with raw materials, generate any required intermediate chemical compounds, and finally generate the desired target molecule.

Additionally, molecules database 114 and synthesis database 116 may also include financial cost information relating to cost of materials, cost of required resources and labor costs involved in the sequence of steps and materials required to construct the desired target composition.

The ability to integrate data from such chemistry databases (i.e., molecules database and synthesis database) to generate a synthesis plan represents one aspect of the invention. Other aspects of the invention relate to ongoing tracking of information based on the synthesis plan and reporting of information during execution of the synthesis plan. Details of processes associated with these aspects and associated user interaction are provided further herein below.

Still further, other aspects of the invention permit the molecule and/or synthesis databases to be searched by various chemical indicia, Such chemical indicia may include chemical terms, symbols and molecular representations. Chemical indicia may be used to search for raw materials involved in projects, intermediate compounds involved in projects and final target molecules involved in projects. Such searches based on chemical indicia may be more useful to scientists and development personnel involved in projects as distinct from financial, marketing and administrative personnel involved in the same projects.

Those of ordinary skill in the art will readily recognize numerous equivalent architectures to that of figure 1 that may benefit from application of features and aspects of the present invention. In particular, any number of organizations within an enterprise may share the information generated and maintained in the form of the project synthesis plan. The particular organizations shown in the exemplary enterprise 2 of figure 1 are therefore merely intended as suggestive of one possible enterprise structure. Still further, those of ordinary skill in the art will recognize that a number of commercially available databases providing molecule information and chemistry synthesis information may be used in conjunction with the features of the present invention. Regardless of the source of such molecule and chemistry procedure information, the present invention integrates such information and shares relevant portions of such information among a number of groups within a chemical development service provider enterprise.

Further clarity of one contrast of certain features and aspects of the present invention may be had in understanding that present solutions for chemical management relate to chemical attributes of compounds, i.e., molecule structure, molecular weight etc. Current solutions to project planning often combine resource planning, costing, activity planning etc. By contrast, features and aspects of the present invention combine such dimensions as chemical information including chemistry and quality assurance, project management information, financial information, etc. in an integrated solution.

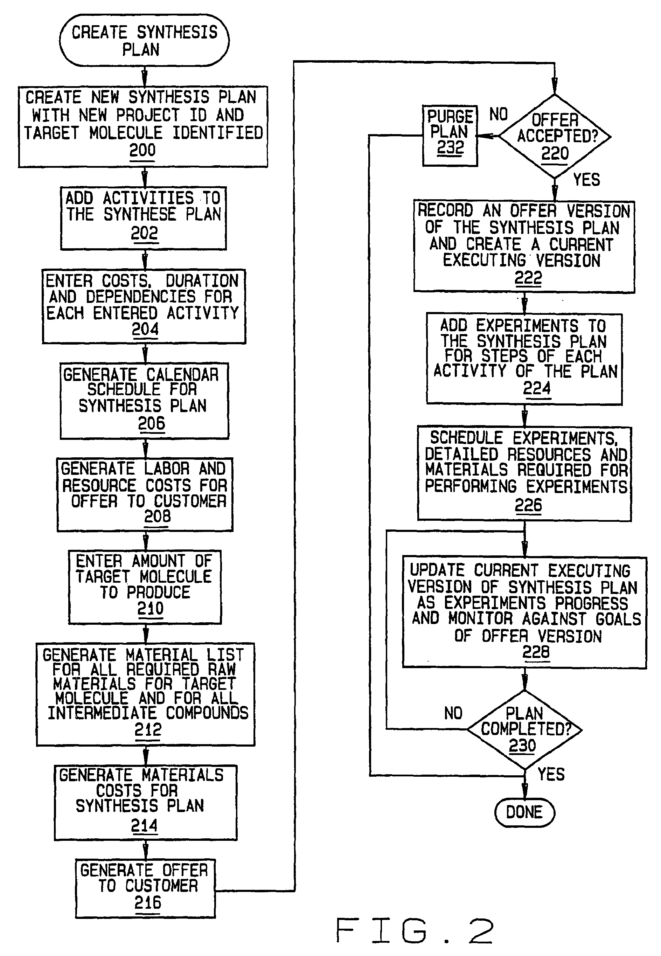

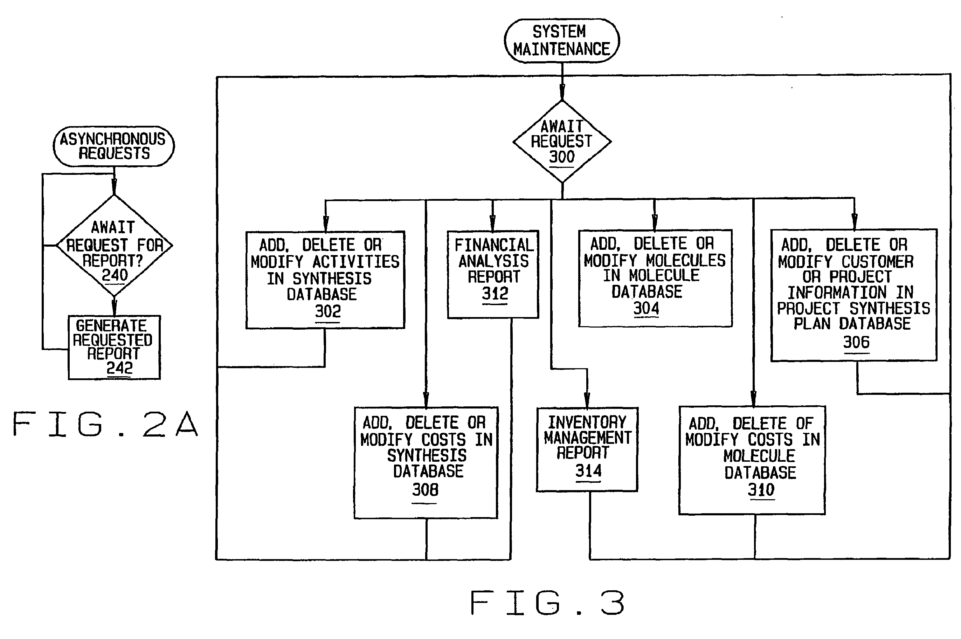

Figure 2 is a flowchart describing a method associated with the present invention for generating and utilizing a project synthesis plan. As noted above, a sales and marketing organization of a custom chemical development service provider may generate such a synthesis plan in response to a request from a customer. Element 200 is first operable to create a new synthesis plan entry with a new project identifier and desired target molecule as specified by the customer. Element 202 is operable to add steps and activities associated with generation of the target module into the newly defined synthesis plan entry. The specific steps and activities associated with generation of the target module may be derived with reference to a molecule database and a synthesis database that, in combination, may defme processes and materials required to generate the desired target module, including intermediate compounds (if any).

Element 204 then determines costs, duration and dependencies associated with each of the entered steps and activities. Costs of resources and materials required in the various steps and activities may be derived with reference to the molecules and synthesis databases. Expected duration for completing each of the identified steps and activities may also be derived with reference to such chemistry databases. Further, the order and dependencies among the various activities and steps may also be derived with reference to such standard chemical databases. For example, activities associated with generating a next intermediate compound must precede steps and activities relating to usage of that intermediate compound.

Element 206 next determines an appropriate calendar schedule to perform the steps and activities described thus far in the newly defmed synthesis plan.
Generating such a calendar schedule for a synthesis plan may require reference to presently defined schedules for other projects being performed within the enterprise. Such scheduling information may be derived with reference to previously entered, presently executing projects within the project synthesis plans database. Various resources within the enterprise may be shared among a plurality of ongoing project development processes and teams. Therefore, calendar scheduling should be performed with reference to such other ongoing projects.

Having so determined an estimated calendar schedule for the new project, element 208 may then determine costs associated with identified labor and other resources associated with the generated schedule. Element 210 is operable to identify the desired amount of the specified target molecule. Although the customer may be a volume manufacturer of such chemical compounds, it may contract with the development service provider for a predetermined initial volume of the specified target molecule. Based on the desired amount of the target molecule, element 212 is next operable to generate a materials list for all required raw materials to generate the desired target molecule as well as all intermediate compounds (if any). Element 214 may then estimate the materials cost and estimated delivery dates for all materials associated with the newly entered synthesis plan.

Having so determined estimated resources, schedule and associated costs, element 216 is next operable to generate an offer to the customer identifying the price for the development of the requested target molecule as well as an estimated schedule for completion of the project to develop the requested target molecule. Generation of such an offer to the customer may be performed through standard software tools to generate a memorandum or other letter format offer automatically from the information entered into the newly defmed synthesis plan. In other words, an offer, in whatever form desired, may be substantially automatically derived from the information stored in the synthesis plans database.

Typically, no further action need be taken with regard to the generated offer until the offer is accepted by the customer as detected by element 220.
If the offer is never accepted by the customer, it may be purged by element 232 from the synthesis plans database or otherwise processed in accordance with typical administrative regulations of the service provider enterprise. If the offer is accepted by the customer, the synthesis plan from which the offer was generated can be locked to save as a baseline reference for ongoing work for the project. This locked, baseline offer synthesis plan may then be used to measure quality, productivity and profitability of the project as work proceeds through implementation.

Element 222 creates a copy of the synthesis plan as accepted by the customer. The original copy may be referred to as the offer version and is retained to be used as a baseline in measuring progress and success of the specified project. The new copy of the synthesis plan may be referred to as the "executable version" or the "executable version of the synthesis plan." The executable version of the synthesis plan may be used going forward as an active repository for information regarding progress of the project. Comparisons may be made between the offer version and the executable version to compare actual progress and costs on the project as compared to the estimates in the offer version. Such comparisons may be informative in tracking financial and other performance criteria for the service provider enterprise.

Element 224 adds detailed "experiments" to the executable version of the synthesis plan for each of the identified activities identified in the synthesis plan. Experiments as used herein refer to details of the actual executed steps, activities, actions, observations, and measures to be performed by an appropriate scientist or engineer within the service provider enterprise. Such experiments document the precise chemistry to be performed for a specified activity or step within the synthesis plan. Recording execution of such experiments in the executable version of the synthesis plan allows more detailed tracking of the precise chemistry involved in producing the desired target molecules. Quality assurance, regulatory compliance and other manufacturing issues may depend on such detailed documentation of the precise chemical procedures followed in producing the target molecule.

Element 226 then generates a more detailed schedule based on the defmed experiments, the precise resources required in each experiments and the precise materials required to complete the defined experiments. With such a detailed schedule now in place, actual processing of the synthesis plan may proceed with confidence in the repeatability and quality of the desired outcome. As the synthesis plan is performed, element 228 is operable to update the executable version of the synthesis plan to indicate progress of the various experiments and to monitor cost goals and schedule goals as documented in the original offer version of the synthesis plan. Updating of the executable version of the synthesis plan may continue until element 230 determines that the entire synthesis plan has been completed thereby completing the project including delivery to the customer.

In parallel with the creation and execution of the synthesis plan, asynchronous requests for particular reports may be generated as shown in elements 240 and 242. Such reports may include, for example, progress reports regarding actual schedule and costs as compared to the original offer version of the synthesis plan as regards schedule and costs or other financial and management related reports. One common example of such a requested report may be in the form of a Gantt chart useful for visually measuring progress in the schedule as compared to the expected schedule documented in the offer version of the synthesis plan. Those of ordinary skill in the art will recognize a wide variety of management reports that may be generated in response to user requests.

The flowchart of figure 2 is intended merely as exemplary of one possible method for creating and executing a synthesis plan in response to the customer requests for producing a target molecule. Those of ordinary skill in the art will recognize a wide variety of similar procedural steps to accomplish the same goal. One aspect of the present invention is based upon the integration of synthesis plans with molecular database information and chemistry synthesis database information to allow multiple organizations within a chemistry service provider enterprise to share common data associated with execution and monitoring of such a synthesis plan on behalf of the customer.

Other aspects of the present invention allow typical administrative tasks to be performed with respect to the various databases maintained in accordance with the present invention. Figure 3 provides a flowchart describing such administrative aspects of the present invention. In particular, element 300 awaits a request from an administrative user within the chemistry service provider enterprise indicating particular administrative needs to be performed. In response to receiving such a request, a selected one of elements 302 through 314 is operable to perform the specific requested administrative tasks. Element 302, for example, is operable to add, delete or modify status or activities relating to chemistry as defined in a synthesis database. As the chemical service provider enterprise evolves through time and experience, additional chemical procedures and activities may be recorded in the synthesis database for possible reuse in a later project. In like manner, element 304 is operable to enable administrative additions, deletions or modifications for molecule information in a molecule database. Element 306 is similarly operable to allow administrative additions, deletions or modifications to basic customer or project information within the project synthesis plans database. Elements 308 and 310 are similarly operable to allow fmancial information associated with the synthesis plan database and molecule database to be updated periodically. The costs of raw materials may change from time to time or costs associated with resources of the enterprise may change from time to time such that an administrative user may update database information relating to costs. Lastly, elements 312 and 314 may be operable to provide special administrative reports relating to, for example, financial analysis of one or more particular projects or relating to inventory control. Financial analysts within such a chemical service provider enterprise may perform analysis relating to profitability of the enterprise or relating to basic financial reporting requirements of the enterprise. In like manner, inventory control managers within the chemical service provider enterprise may review present inventory as it relates to ongoing projects for purposes of maintaining a proper flow of raw materials through the enterprise. Those of ordinary skill in the art will readily recognize numerous other administrative and reporting tasks that may be desirable or beneficial within the scope of the present invention. Figure 3 is therefore merely intended as representative of typical administrative and reporting tasks that may be performed within such a chemical service provider enterprise.

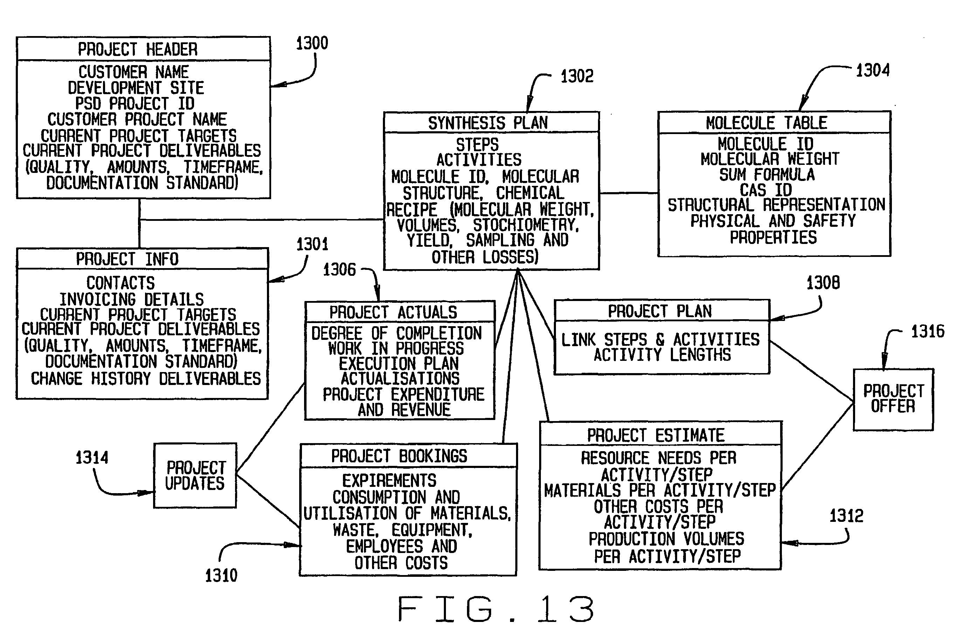

Figure 13 is a diagram of exemplary database structures that may be employed in implementing features of the present invention. The structures depicted in figure 13 are not intended to represent specific file, table or record structures of any particular database or file management system. Rather, the elements of figure 13 are intended to suggest possible relationships between broad categories of information. A database structure associated with features and aspects of the invention may be principally indexed by records stored in a project headers structure 1300.
Information regarding the customer and the project may be stored in such a table. Additional details regarding a particular project may be stored in a related project info structure 1301. A project identifier may be used to relate the two structures. Using such a project identifier, a corresponding synthesis plan may be located in a synthesis plan structure 1302. The synthesis plan entries may reference particular chemical compounds and may refer to entries in a molecule table structure 1304. In addition, synthesis plan information in structure 1302 may reference other information relating to planned steps, activities, personnel, resource and materials in structures 1308 and 1312. Actual progress of a project may be referenced through reference to structures
1306 and 1310. These structures may store information regarding actual progress of the project with respect to personnel hours, materials and sequencing through the desired activities and steps.

Offers to a customer may be stored in structure 1316 and reference information in the project planning information in structures 1308 and 1312. Project status updates may be stored in structure 1314 and may reference information in actual tracking information of the project in structures 1306 and 1310.

Specific database or file structures to implement such data relationships will be readily apparent to those of ordinary skill in the art as a matter of design choice. Further, such design choices may group information differently than depicted in figure 13 to improve the normalization of information in the database as implemented. Such normalization techniques are well known to those of ordinary skill in the art to reduce duplication of data and the errors that may arise therefrom.

In addition, customer specific information may also be stored in a separate table to permit further normalization of the projects table structure.

Those of ordinary skill in the art will readily recognize that the database structures described with reference to figure 13 are intended merely as exemplary of typical database structures that may be used in implementing features of the present invention. Numerous equivalent database structures or other indexed file structures will be readily apparent to those of ordinary skill in the art.

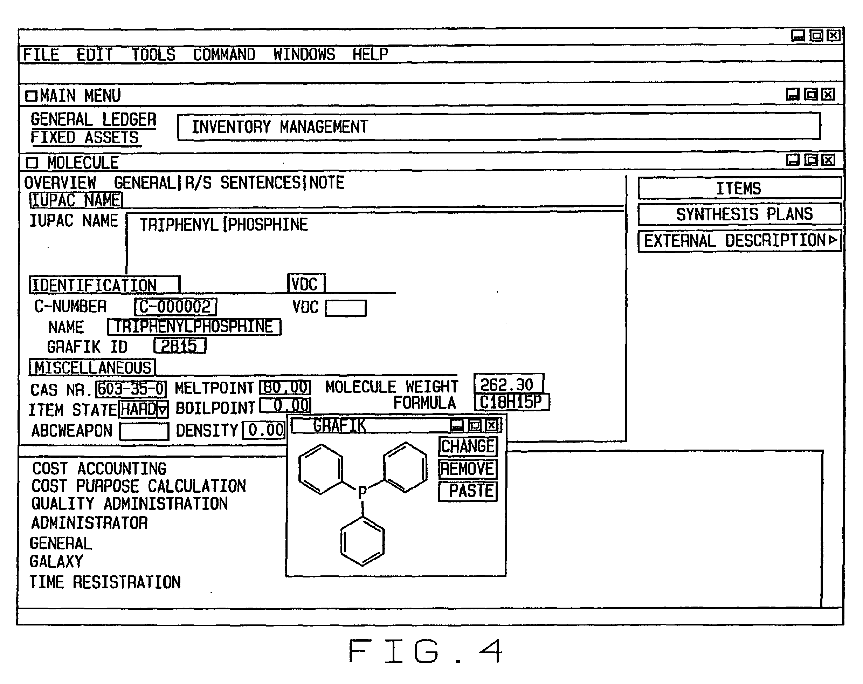

Figures 4 through 12 and 14 through 20 depict exemplary user display screens for interacting with a user applying the features and aspects of the present invention. Typical user interaction may include, for example, entry of a new customer into the proj ect synthesis plans database and entry of a new project in the plans database. Other exemplary user interactions involve steps to defme activities and materials associated with a particular target material for a particular customers project. Those of ordinary skill in the art will recognize the following user display screens as merely exemplary of some common user interactions associated with the present invention. Numerous other user interactions will be readily apparent to those of ordinary skill in the art.

Further, the style and structure of these exemplary user display screens are intended merely as exemplary of possible screen layouts and structures. These particular exemplary screen designs are typical of user interface display screens generated using Microsoft. NET Business Solutions development tools. As another design choice, features and aspects of such display screens may be implemented in any programming language (such as C++) operable on any computer platform (such as a Windows PC or a UNIX workstation) using other standard, commercially available display design application programming interfaces. Those of ordinary skill in the art will recognize a wide variety of similar screen layout designs and user interaction structures that may be developed using the Microsoft ,NET Business Solutions framework as well as other common application development suites.

Figure 4 shows an exemplary display screen used to track molecules and chemical compounds known within the service provider. Every known molecule is defined in terms of its structure, molecular weight, chemical formula, and other information. All projects performed by the service provider on behalf of a customer should preferably be based on known chemistry, compounds and reactions. This help ensure desired levels of safety within the service provider and desired levels of quality in the products generated by the service provider. Once a quality assurance organization within the service provider has evaluated the process and materials involved with a new product, projects may be planned using the newly reviewed material. Though such safety and quality goals are usually important to a service provider, the present invention does not require such quality review. Rather, a new project on behalf of a customer may for the first time introduce new procedures and processes to the service provider. Such options will be readily apparent to those of ordinary skill in the art.

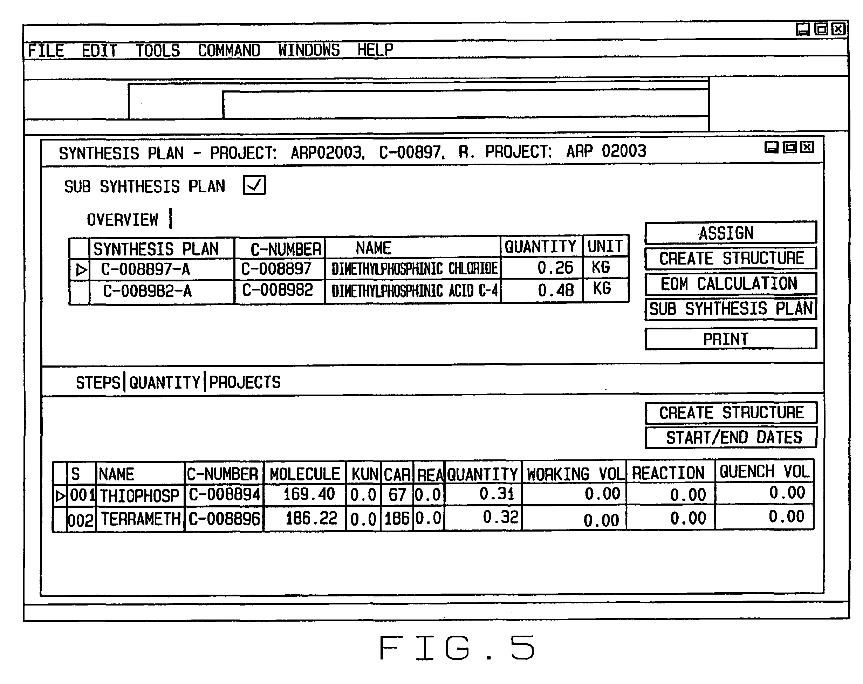

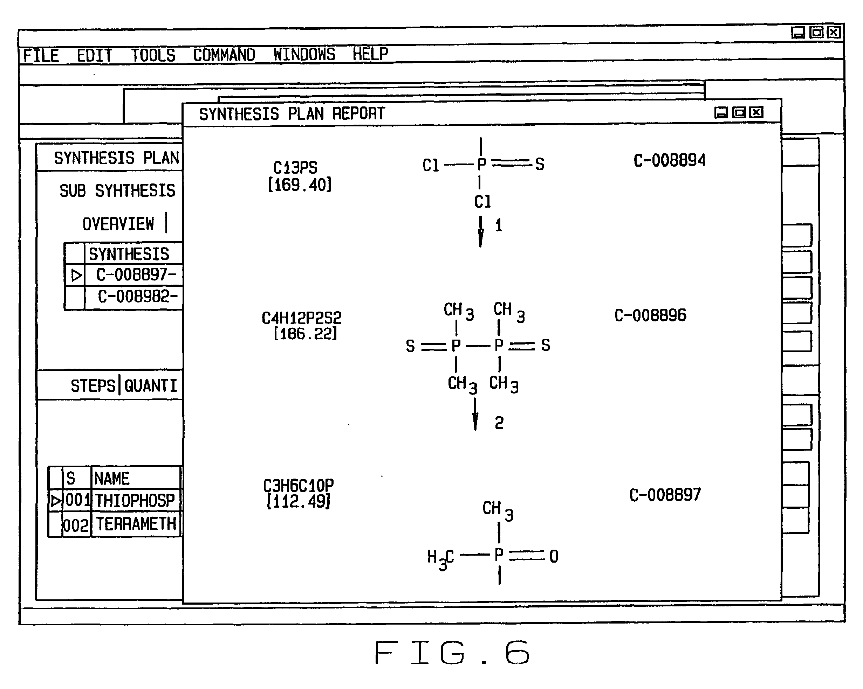

Figure 5 shows an exemplary display screen for creating a new synthesis plan for a project. Once all molecules/compounds required to create a desired target compound are defined and approved for use, a synthesis plan may be constructed to plan for resources required to produce the target material. Chemical materials are often referred to by their respective CAS Numbers (Chemical Abstract Society). The CAS number may be used as a consistent shorthand notation for a defined chemical compound. Figure 6 shows an exemplary synthesis plan to produce two desired compounds commonly referred to by CAS number C-008897 and C-008982. The project to produce the material has been named ARP02003. An upper portion of the displayed table shows the desired target compounds and the desired quantity of each material. Two compounds are to be produced under this project. A first is .48 kg of C-008982 and a second producing .25 kg of C-008897. With one of the target molecules selected (C-008897), the lower portion of the exemplary screen of figure 5 shows the steps associated with generation of the defined target molecule. Specifically, step "0001" indicates use of .31 kg of material C-008894 and step "0002" indicates use of ,23 kg of C-008896 produced from step "0001" to generate the desired target molecule (C-008897). Figure 6 shows the steps of the same exemplary synthesis plan graphically reporting the molecular structures and reactions involved in the steps. In particular, the display shows step 0001 using C-008894 to produce C-008896. Step 0002 then uses the intermediate compound C-008896 to produce the desired target material C-008897.

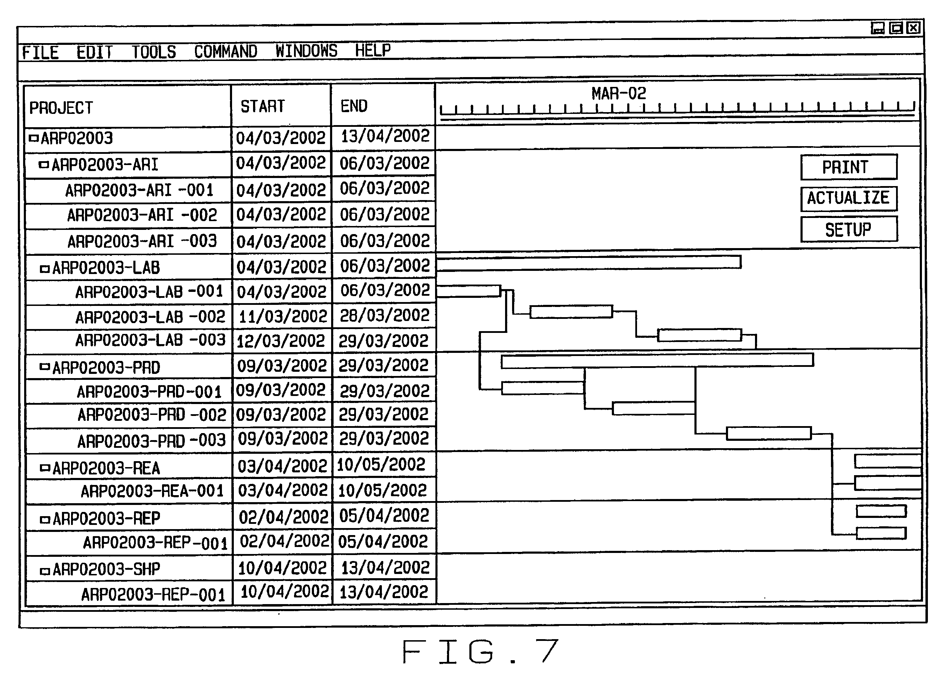

Figure 7 shows the same exemplary project synthesis plan with dates associated with each step and activity. The project ARP02003 is shown on the left as a tree structure with each activity broken out at a next level of the tree. In particular, ARP02003.ART is generally followed by ARP02003.LAB, in turn followed by ARP02003.PRD, ARP02003.REA, ARP02003.REP and ARP02003.SHP. Each activity indicates its associated steps and intermediate compounds such as ARP02003.LAB.C-008897-A.0001, ARP02003.LAB.C-008897-A.0002, and ARP02003.LAB.C-008982-A.0001. The various steps ad activities are shown laid out on a timeline as a Gantt chart linking critical path elements. As a project is underway, the Gantt chart may be annotated by features and aspects of the invention to indicate progress in the development.

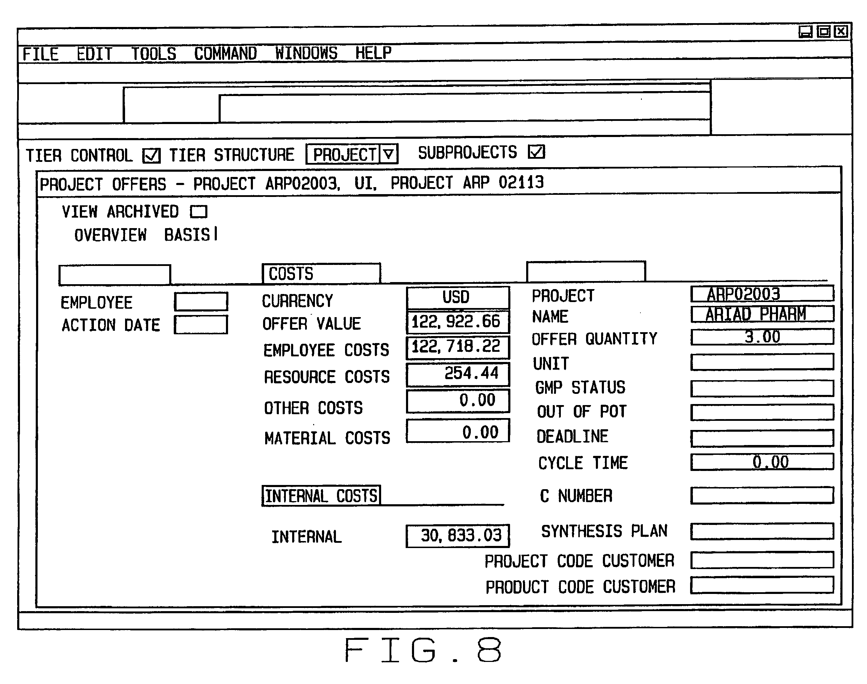

Figure 8 is an exemplary screen display showing an offer generated for a specified project. The offer may be generated by aspects of the invention to provide a price and/or scheduling quote to a customer for production of the specified material. A quote may be broken down to indicate costs associated with personnel, with materials cost, and with costs of resources used in the process. Other internal costs or margins may be added to such an offer to be presented to a customer.

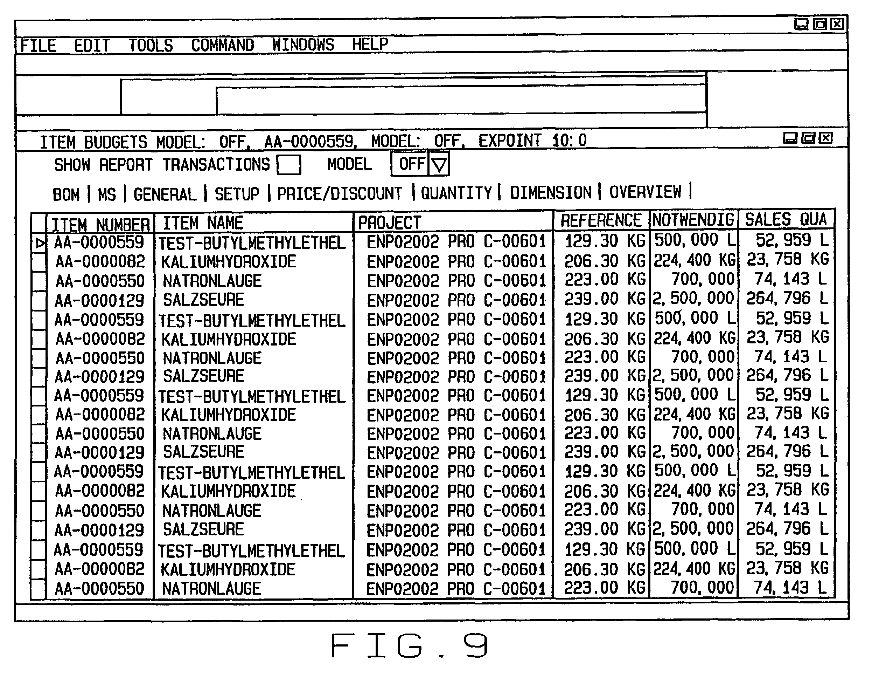

As noted above, a project is typically commenced only when a customer accepts such an offer. Figure 9 shows an exemplary display screen providing a user with materials planning information for a project. A bill of materials for exemplary project (ENP02002) is shown. Quantities required for each item to produce the desired target or intermediate molecule are listed. Such a list may be provided to a customer as support for materials portions of a price quote or may be used in inventory control aspects of the invention. In inventory control, aspects of the invention will notify an appropriate materials manager user to alert that person to the upcoming need for materials on a new project. As the project progresses, actual material usage may be tracked against the projected use.

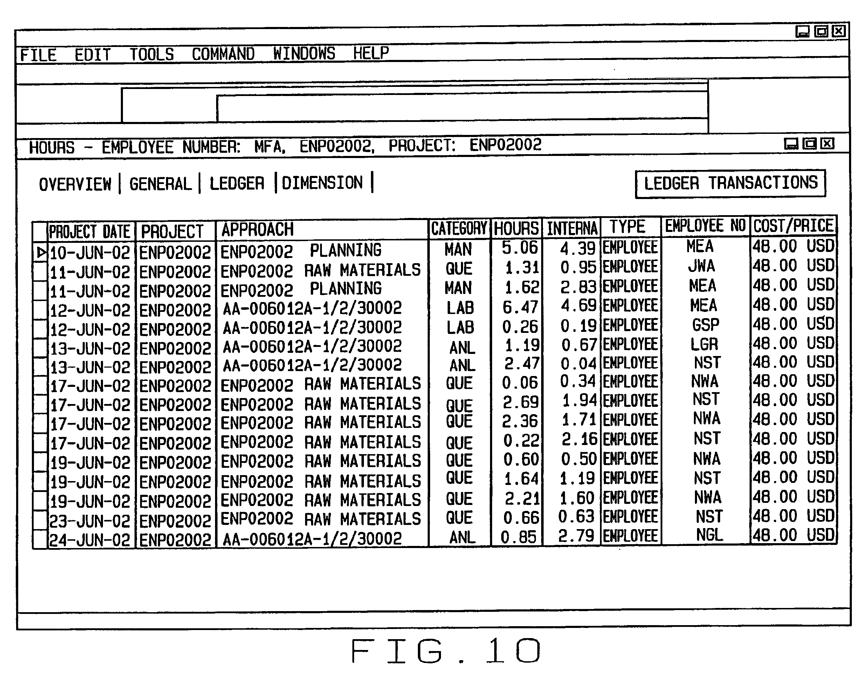

Figure 10 provides an exemplary display screen for summarizing expected employee hours required for a project. Project managers may then review the anticipated personnel needs and schedule accordingly. As for materials noted above, actual personnel hours used on a project may be tracked against projected hours as the project progresses. Financial management within the service provider may also review such information to determine actual costs, to determine work in progress, revenue projections, etc.

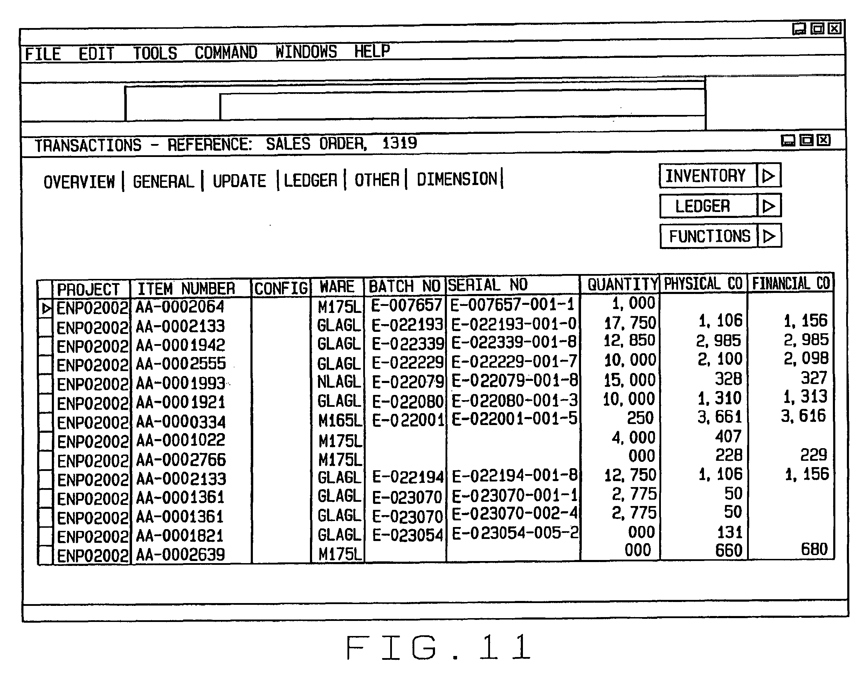

Figure 11 provides an exemplary display screen for item or material bookings. As materials are actually consumed in a project, booking entries are generated from the materials management. These records include information such as material manufacturer, costs, batch numbers, etc. to permit tracking of a compound for quality and other purposes. Financial and materials management entities within the service provider may also refer to this information for their respective functions.

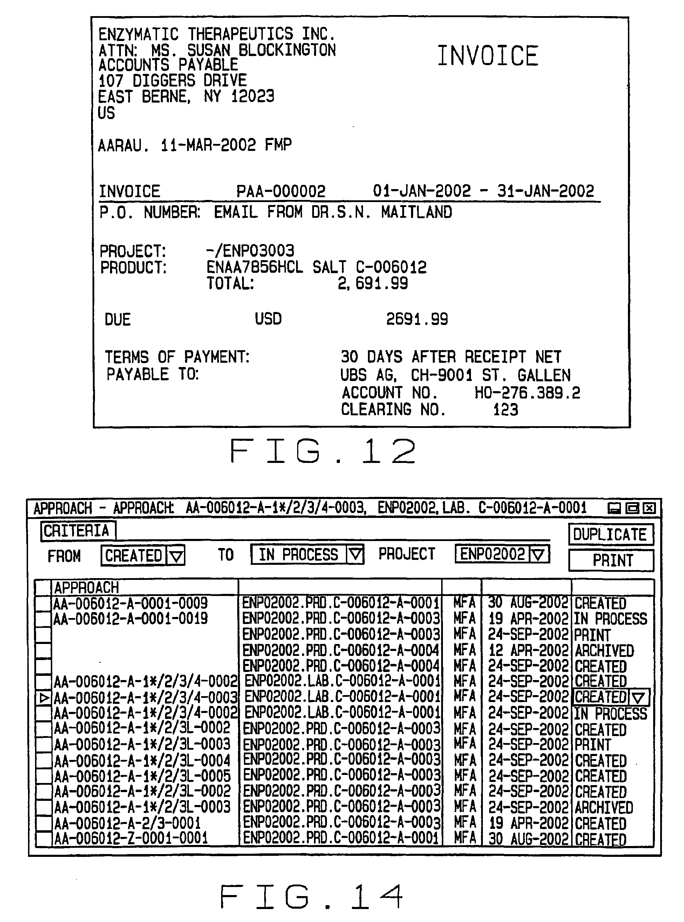

The exemplary display screen of figure 12 shows a sample customer invoice generated from the information within the service provider's systems upon completion of a project. With information tightly integrated as discussed herein, all aspects of chemical product development from initial offer generation, through project execution to final invoicing may be performed.

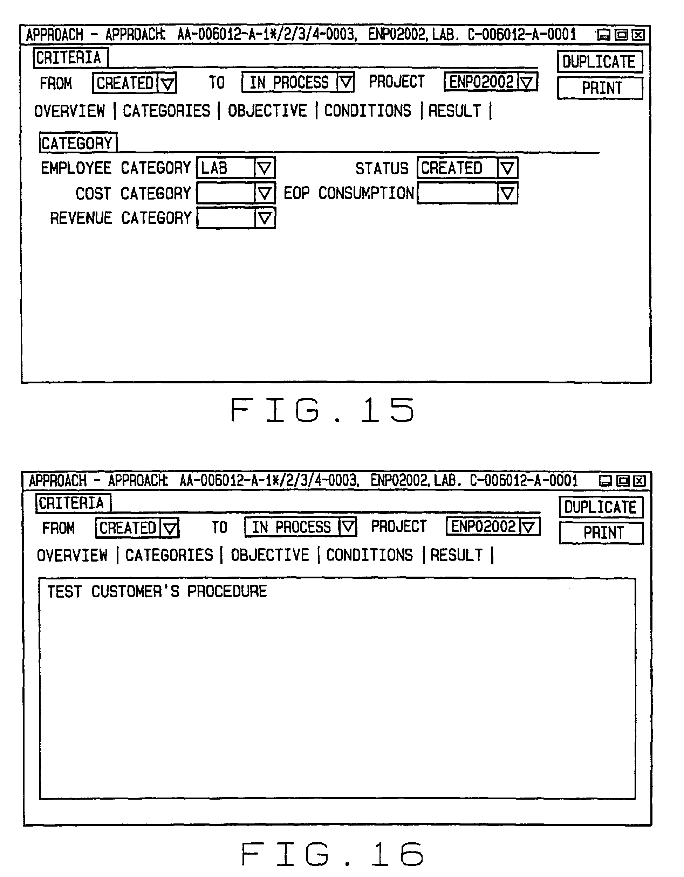

Figures 14 through 20 are additional exemplary display screens relating to user interaction for experiments (also referred to in the figures as "approaches"). As noted above, experiments (or "approaches") refer to details of the actual executed steps, activities, actions, observations, and measures to be performed by an appropriate scientist or engineer within the service provider enterprise. Such experiments document the precise chemistry to be performed for a specified activity or step within the synthesis plan. Recording execution of such experiments in the executable version of the synthesis plan allows more detailed tracking of the precise chemistry involved in producing the desired target molecules.

Figure 14 shows an exemplary Overview screen with a list of experiments associated with an identified project (synthesis plan). Each particular experiment (labeled "Approach") may be identified by a unique identifier. The associated project may be listed along with the responsible chemist. Various dates and status associated with the experiments may also be presented in the tabular listing. As shown in figure 14, a drop-down menu or other user interface structure may be employed to permit a user to update the status of an identified experiment. By selecting one of the experiments listed in the table, additional information may be presented in subsequent display. Such subsequent displays may include, for example, those indicated by ''tabs'' in the display of figure 14 (i.e., Categories, Objective, Conditions and Result display screens for display of particular attributes associated with the selected experiment).

Figure 15 is an exemplary display screen to show category attributes associated with a selected experiment. Each experiment may include categories to group related experiments according to like attribute values. Such categories may include, for example, the employee type responsible for the experiment ("Employee Category"), a cost identifier for categorizing the costs associated with the experiment ("Cost Category"), a revenue identifier for associating the revenue with a particular business group ("Revenue Category"). Other fields may be displayed on the category display screen including, for example, the present status of the selected experiment.

A description of the objective of a selected experiment may be presented on a display screen such as shown in figure 16. The objective of an experiment may be entered by a user initially when a project synthesis plan is defined and the experiments are added to the plan. This exemplary display screen allows the user (i.e., engineer or chemist) to view and update the objective of the selected experiment as the project evolves.

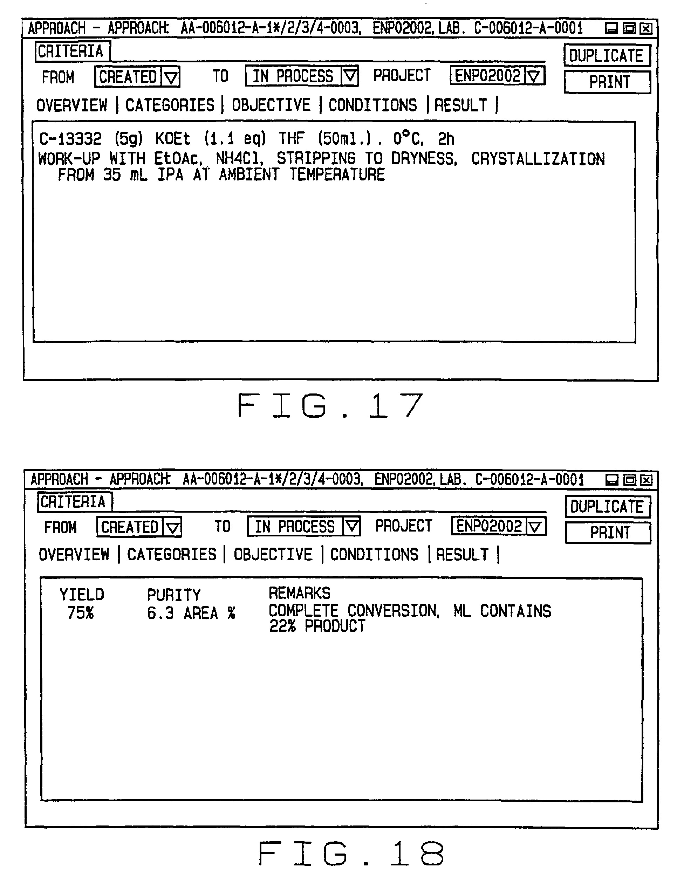

Environmental conditions and other external aspects necessary to perform the selected experiment may be presented to the user in a display such as that of figure 17. The chemist or engineer may refer to the conditions display to verify proper environment for performing the selected experiment. In addition, the chemist may update the conditions display to record the actual conditions present when the experiment is performed.

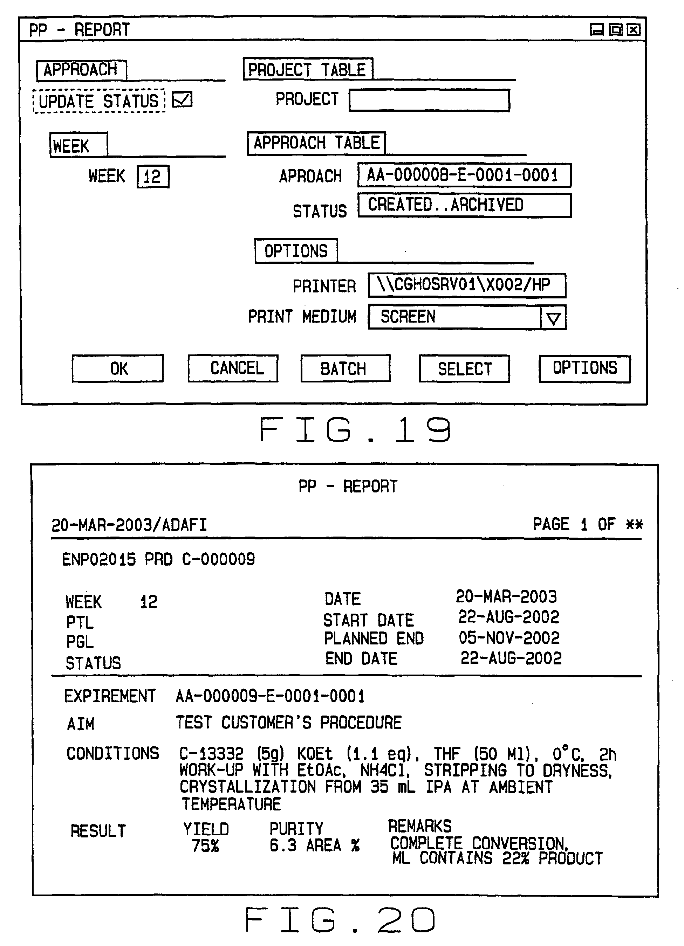

The results of an experiment performed by an appropriate chemist or engineer may be recorded and reviewed in a display such as that of figure 18. By clicking the print button of such a display, the user may generate a stylized report of such results for recordation in paper form or for presentation to customers or regulators. Figure 19 shows an exemplary dialog that may be presented in response to the print button for options in generating such a report. Lastly, figure 20 shows an exemplary report generated by such a request to provide summary and detailed information regarding performance of a selected experiment.

Those of ordinary skill in the art will readily recognize a wide variety of equivalent screen displays as well as additional screen displays useful for interacting with a user for purposes of creating, modifying or executing the project synthesis plan. In particular, other display screens will be readily apparent to those of ordinary skill in the art intended to aid the user in creating a bill of materials for the required materials associated with the project, for defining and conducting experiments associated with actual execution of the defined synthesis plan, etc. The screen displays of figures 4 through 12 and 14 through 20 are therefore intended merely as exemplary of typical displays useful in performing the functions and implementing the structures of the present invention.

While the invention has been illustrated and described in the drawings and foregoing description, such illustration and description is to be considered as exemplary and not restrictive in character, One embodiment of the invention and minor variants thereof have been shown and described. Protection is desired for all changes and modifications that come within the spirit of the invention. Those skilled in the art will appreciate variations of the above-described embodiments that fall within the scope of the invention. As a result, the invention is not limited to the specific examples and illustrations discussed above, but only by the following claims and their equivalents.

## Claims

1. A method for chemical compound development project management comprising:
providing a molecule database wherein records of the molecule database include molecular information regarding reactants and products of the synthesis steps required to produce an identified target molecule;
providing a synthesis database wherein records of the synthesis database include activity information regarding activities to transition between the synthesis steps of the target molecule synthesis; and
deriving information regarding a chemical compound development project from the molecule database and from the synthesis database.

2. The method of claim 1 wherein the step of deriving includes:
identifying, from the molecule database, materials required in production of the target molecule.

3. The method of claim 2 wherein the step of deriving further includes:
generating a bill of materials identifying quantities of all materials required in production of the target molecule.

4. The method of claim 1 wherein the step of deriving includes:
identifying, from the synthesis database, resources required in production of the target molecule.

5. The method of claim 4 wherein the step of deriving further includes:
identifying, from the synthesis database, activities associated with the identified resources.

6. The method of claim 5 wherein the step of deriving further includes:
generating a GANTT chart scheduling the identified resources and the identified activities in production of the target molecule.

7. The method of claim 1 wherein the step of deriving includes:
identifying, from the molecule database, materials required in production of the target molecule;
identifying, from the synthesis database, resources required in production of 5 the target molecule;
identifying, from the synthesis database, costs associated with the identified materials and associated with the identified resources; and
generating an estimated project cost from the identified costs.

8. The method of claim 1 wherein the step of deriving includes:
searching the molecule database or the synthesis database using chemical indicia to retrieve information regarding a chemical compound development project.

9. A method for chemical compound development project management comprising:
providing a molecule database wherein records of the molecule database include molecular information regarding reactants and products of the synthesis steps required to produce an identified target molecule;
providing a synthesis database wherein records of the synthesis database include activity information regarding activities to transition between the synthesis steps of the target molecule synthesis; and generating a synthesis plan for a target molecule using molecular information from the molecule database and using activity information from the synthesis database,

10. The method of claim 9 wherein the step of generating includes:
identifying, from the molecule database, all intermediate molecules required in production of the target molecule.

11. The method of claim 10 wherein the step of identifying all intermediate molecules includes:
identifying materials required for production of all intermediate molecules and production of the target molecule.

12. The method of claim 11 wherein the step of generating further includes:
identifying, from the synthesis database, activities required for production of all intermediate molecules and for production of the target molecule.

13. The method of claim 12 wherein the step of generating further includes:
identifying, from the synthesis database, resources required for production of all intermediate molecules and for production of the target molecule.

14. The method of claim 13 wherein the step of generating further includes:
identifying, from the molecule database and from the synthesis database, costs associated with the identified materials and with the identified resource and with the identified activities required for production of all intermediate molecules and for production of the target molecule.

15. The method of claim 9 further comprising:
estimating total costs to develop the target molecules; and
generating an offer to develop the target molecule for a customer wherein the offer includes a price derived from the estimated total cost.

16. The method of claim 15 further comprising:
receiving acceptance from the customer to develop the target molecule in accord with the offer; and
adding experiments to the synthesis plans to schedule resources required to develop the target molecule.

17. The method of claim 16 further comprising:
tracking progress of the development of the target molecule based on progress of the experiments.

18. A system for target chemical compound development comprising:
a molecule database including information regarding intermediate chemical compounds required to produce the target chemical compound;
a synthesis database including information regarding steps required to transition between the synthesis steps of the target molecule synthesis; and
a chemical compound development management system for generating project synthesis plans integrating information from both the molecule database and the synthesis database.

19. A system for chemical compound development project management comprising:
means for providing a molecule database wherein records of the molecule 10 database include molecular information regarding reactants and products of the synthesis steps required to produce an identified target molecule;
means for providing a synthesis database wherein records of the synthesis database include activity information regarding activities to transition between the synthesis steps of the target molecule synthesis; and
means for deriving information regarding a chemical compound development project from the molecule database and from the synthesis database.

20. The system of claim 19 wherein the means for deriving includes:
means for identifying, from the molecule database, materials required in production of the target molecule.

21. The system of claim 20 wherein the means for deriving further includes:
means for generating a bill of materials identifying quantities of all materials required in production of the target molecule.

22. The system of claim 19 wherein the means for deriving includes:
means for identifying, from the synthesis database, resources required in production of the target molecule.

23. The system of claim 22 wherein the means for deriving further includes:
means for identifying, from the synthesis database, activities associated with the identified resources.

24. The system of claim 23 wherein the means for deriving further includes:
means for generating a GANTT chart scheduling the identified resources and the identified activities in production of the target molecule.

25. The system of claim 19 wherein the means for deriving includes:
identifying, from the molecule database, materials required in production of the target molecule;
identifying, from the synthesis database, resources required in production of the target molecule;
identifying, from the synthesis database, costs associated with the identified materials and associated with the identified resources; and
generating an estimated project cost from the identified costs.

26. The system of claim 19 wherein the means for deriving includes:
means for searching the molecule database or the synthesis database using chemical indicia to retrieve information regarding a chemical compound development project.

27. A computer readable storage medium tangibly embodying program instructions implementing a method for chemical compound development project management, the method comprising:
providing a molecule database wherein records of the molecule database include molecular information regarding reactants and products of the synthesis steps required to produce an identified target molecule;
providing a synthesis database wherein records of the synthesis database include activity information regarding activities to transition between the synthesis steps of the target molecule synthesis; and
deriving information regarding a chemical compound development project from the molecule database and from the synthesis database.

28. The medium of claim 27 wherein the method step of deriving includes:
identifying, from the molecule database, materials required in production of the target molecule.

29. The medium of claim 28 wherein the method step of deriving further includes:
generating a bill of materials identifying quantities of all materials required in production of the target molecule.

30. The medium of claim 27 wherein the method step of deriving includes:
identifying, from the synthesis database, resources required in production of the target molecule.

31. The medium of claim 230 wherein the method step of deriving further includes:
identifying, from the synthesis database, activities associated with the identified resources.

32. The medium of claim 31 wherein the method step of deriving further includes:
generating a GANTT chart scheduling the identified resources and the identified activities in production of the target molecule.

33. The medium of claim 27 wherein the method step of deriving includes:
identifying, from the molecule database, materials required in production of the target molecule;
identifying, from the synthesis database, resources required in production of the target molecule;
identifying, from the synthesis database, costs associated with the identified 25 materials and associated with the identified resources; and
generating an estimated project cost from the identified costs.

34. The medium of claim 27 wherein the method step of deriving includes:
searching the molecule database or the synthesis database using chemical indicia to retrieve information regarding a chemical compound development project.

35. An interface for integrating various information management functions, the interface comprising computer readable program code devices for:
receiving a request to develop materials and procedures for a synthesis plan appropriate for manufacturing a particular desired chemical compound;
creating a new project synthesis plan entry in the project synthesis plan database correlating to the desired chemical compound;
accessing a molecule database to retrieve molecular information regarding reactants and products of synthesis steps required to produce the desired chemical compound;
accessing a synthesis database to retrieve activity information regarding activities to transition between the synthesis steps of the desired chemical compound synthesis; and
deriving information regarding the chemical compound project synthesis plan from information retrieved from the molecule database and the synthesis database and adding steps and activities to the project synthesis plan.

36. The interface as recited in claim 35 where the readable program code devices for deriving information includes identifying from the molecule database, material required in production of the desired chemical compound.

37. The interface as recited in claim 36 where the readable program code devices for deriving information includes generating a bill of materials identifying quantities of all materials required in production of the desired chemical compound.

38. The interface as recited in claim 35 where the readable program code devices for deriving information includes identifying from the synthesis database, resources required in production of the desired chemical compound.

39. The interface as recited in claim 38 where the readable program code devices for deriving information includes identifying from the synthesis database activities associated with identified resources.

40. The interface as recited in claim 39 where the readable program code devices for deriving information includes generating a GANTT chart scheduling the identified resources and the identified activities in production of the desired molecule.

41. A computer readable medium containing a data structure for storing data relevant to the management of chemical compound development and for integrating and sharing data to derive a project synthesis plan, the data structure comprising:
a project header data structure containing basic project information including a project identifier and a desired chemical compound; and
a synthesis plan data structure containing synthesis plans that can be correlated by the project identifier to the project header data structure and correlated by molecule identifiers and structures that reference to molecule data structures.

42. The computer readable medium containing a data structure as recited in claim 42 where the synthesis plan data structure contains a project plan reference for referencing a project plan data structure containing specific steps and activities for developing the desired chemical compound ,

43. The computer readable medium containing a data structure as recited in claim 42 where the synthesis plan data structure contains a project estimate reference for referencing a project estimate data structure containing resource needs for each step and activity and material needs.
